# EUROPEAN PATENT APPLICATION

(11) **EP 3 078 663 A1**
(43) Date of publication of application: **12.10.2016**
(21) Application number: 15162960.7
(22) Date of filing: 09.04.2015
(51) Int. Cl.: C07D 471/04, A61K 31/519, A61P 35/00

(54) **MODIFIED PARTICLES OF PALBOCICLIB**

(71) Applicant: SANDOZ AG, 4056 Basel (CH)
(72) Inventor: The designation of the inventor has not yet been filed
(74) Representative: Kluschanzoff, Harald

(57) **Abstract**

The invention relates to crystalline free base of palbociclib having medium specific surface area, to flake- and/or plate-like primary particles of Palbociclib and to a modified salt break procedure for the preparation thereof. The invention is also directed to pharmaceutical compositions comprising an effective amount of palbociclib and a pH modifier and to the use of said pharmaceutical compositions as medicament, in particular for the treatment of cancer.

## Description

### FIELD OF THE INVENTION

The invention relates to crystalline free base of palbociclib having medium specific surface area, to flake- and/or plate-like primary particles of Palbociclib and to a modified salt break procedure for the preparation thereof. The invention is also directed to pharmaceutical compositions comprising palbociclib and a pH modifier and to the use of said pharmaceutical compositions as medicament, in particular for the treatment of cancer.

### BACKGROUND OF THE INVENTION

6-Acetyl-8-cyclopentyl-5-methyl-2-(5-piperazin-1-yl-pyridin-2-ylamino)-8*H*-pyrido[2,3-*d*]pyrimidin-7-one (international non-proprietary name: palbociclib) represented by chemical structure (I) 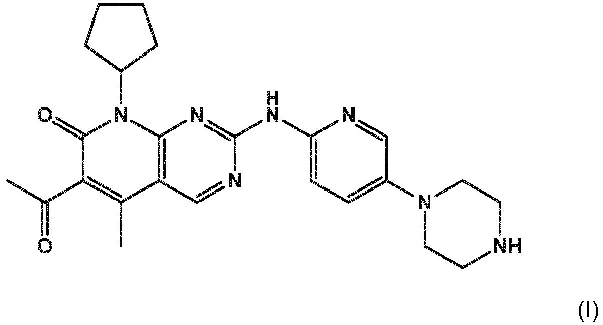 is a potent and selective inhibitor of cyclin-dependent kinases 4 and 6 (CDK4 and CDK6).

Palbociclib and pharmaceutically acceptable salts thereof are disclosed in WO 2003/062236 A1 which describes the preparation of palbociclib as its hydrochloride salt. WO 2005/005426 A1 describes the preparation of palbociclib free base and various mono- and di-acid addition salts thereof including various polymorphic forms of the isethionate salt.

WO 2014/128588 A1 teaches that palbociclib free base provided by salt break procedures, e.g. as described in example 4 of WO 2005/005426 A1, was highly static prone and formed small primary particles, which agglomerated into large, hard agglomerates that were difficult to disperse by sieving and were unsuitable for further development (see page 2, lines 4 to 7). Therefore, WO 2014/128588 A1 provides palbociclib having larger primary particles and reduced specific surface area, which, according to the applicant, demonstrates improved physicochemical and manufacturability properties.

Due to its low solubility and high permeability palbociclib can be put into class II of the Biopharmaceutical Classification System (BCS). When administering such low soluble substances orally, the dissolution rate is the limiting factor during drug absorption. A decrease in specific surface area results in slower dissolution because of less solid-solvent interaction. Hence, although palbociclib free base provided in WO 2014/128588 A1 seems to overcome the technical hurdles with regards to pharmaceutical processing, there is still a need for improving its dissolution rate which has a big impact on the bioavailability. Moreover, when repeating the method provided in WO 2014/128588 A1 it was found that palbociclib was obtained with low yield.

Moreover palbociclib presents a pH-dependent solubility, which decreases as the pH increases. In people having a pH higher than normal in the stomach, the dissolution of Palbociclib is thus impaired and this leads to a decreased bioavailability of the drug.

Hence, aim of the invention was to provide crystalline palbociclib free base which on the one hand has powder properties allowing for straight forward pharmaceutical processing and on the other is characterized by a high dissolution rate and consequently improved bioavailability. Another object of the invention is to provide Palbociclib particles with lower sticking tendency during processing and better flow properties. Yet another object of the invention is to provide palbociclib formulations that are more convenient to process and that show improved bioavailability also in people having a pH higher than normal in the stomach. Another object of the invention was to provide a process which delivers palbociclib in high yield and purity, while at the same time allowing an easy filtration of the active ingredient.

### SUMMARY OF THE INVENTION

The present invention relates to crystalline free base of palbociclib having medium specific surface area and to a modified salt break procedure for the preparation thereof, which delivers palbociclib in high yield and purity and with improved filterability. Palbociclib free base of the invention contains flake- and/or plate-like primary particles and consequently on the one hand shows powder properties viable for pharmaceutical processing and on the other hand ensures fast dissolution and therefore optimal bioavailability. In addition, the present invention is directed to pharmaceutical compositions comprising palbociclib and a pH modifier, preferably crystalline palbociclib free base having medium specific surface area and to the use of said pharmaceutical compositions as medicament, in particular for the treatment of cancer.

### Definitions

In the context of the present invention the following abbreviations have the indicated meaning, unless explicitly stated otherwise:
- PXRD: powder X-ray diffraction/ diffractogram
- SSA: specific surface area
- RT: room temperature

As used herein, the terms "API" or "active pharmaceutical ingredient" or "palbociclib" refer to the free base of 6-acetyl-8-cyclopentyl-5-methyl-2-(5-piperazin-1-yl-pyridin-2-ylamino)-8*H-*pyrido[2,3-*d*]pyrimidin-7-one.

As used herein, the term "primary particles" refers to individual API crystals.

The term "plates" as used herein when talking about crystal habit or particle shape refers to flat, tabular crystals having similar breadth and width, which are thicker than flakes.

The term "flake" as used herein when talking about crystal habit or particle shape refers to thin, flat crystals that have similar breadth and width, which are thinner than plates.

The term "equant" as used herein when talking about crystal habit or particle shape refers to crystals which are equi-dimensional, such as cubes or spheres.

The term "needle" as used herein when talking about crystal habit or particle shape refers to acicular, thin and highly elongated crystals having similar width and breadth.

The term "columns" or "columnar" as used herein when talking about crystal habit or particle shape refers to elongated, prismatic crystals having greater width and thickness than needles.

Such crystal habit definitions are consistent with those usually used in the art, e.g. see "Polymorphism in the Pharmaceutical Industry" edited by Rolf Hilfiker (Wiley-VCH, 2006); Chapter 7, Light Microscopy (Gary Nichols).

As used herein the term "room temperature" indicates that the applied temperature is not critical and that no exact temperature value has to be kept. Usually, "room temperature" is understood to mean temperatures in the range of from 15 to 25 °C [see e.g. European Pharmacopoeia 8.3, 1.2 (2015)].

The term "essentially the same" with reference to PXRD means that variabilities in positions and relative intensities of reflections are to be taken into account. For example, a typical precision of the 2-Theta values is in the range of ± 0.2° 2-Theta. Thus, a reflection that usually appears at 22.5° 2-Theta for example can appear between 22.3° and 22.7° 2-Theta on most X-ray diffractometers under standard conditions. Furthermore, one skilled in the art will appreciate that relative intensities of the reflections will show inter-apparatus variability as well as variability due to degree of crystallinity, preferred orientation, sample preparation and other factors known to those skilled in the art and should be taken as qualitative measure only.

The term "form A" as used herein refers to a certain crystalline form of palbociclib free base. Form A is characterized by having a PXRD comprising peaks at 2-Theta angles of (5.0 ± 0.2)°, (7.9 ± 0.2)°, (10.2 ± 0.2)°, (11.5 ± 0.2)°, (22.5 ± 0.2)°, when measured at room temperature with Cu-Kalpha_{1,2} radiation having a wavelength of 0.15419 nm.

The term pH modifier as used herein refers to substance with acidic or basic properties that can influence the pH of the mixture or the composition to which it is added.

The term "mother liquor" as used herein refers to the solution remaining after crystallization of a solid.

As used herein the term "cancer" includes both solid tumors and hematological malignancies. Cancers include, but are not limited to, breast cancer, ovarian cancer, cervical cancer, endometrial cancer, prostate cancer, testicular cancer, pancreatic cancer, esophageal cancer, head and neck cancer, bladder cancer, lung cancer (e.g. adenocarcinoma, non-small-cell lung cancer and small-cell lung cancer), bone cancer (e.g. osteosarcoma), colon cancer, rectal cancer, thyroid cancer, brain and central nervous system cancers, glioblastoma, neuroblastoma, neuroendocrine cancer, rhabdoid cancer, keratocanthoma, epidermoid cancer, seminoma, melanoma, sarcoma (e.g. lipacarcoma), bladder cancer, liver cancer (e.g. hepatocellular carcinoma), kidney cancer (e.g. renal cell carcinoma), myeloid disorders (e.g. acute myeloid leukemia, chronic myeloid leukemia, myelodysplastic syndrome and promyelocytic leukemia) and lymphoid disorders (e.g. leukemia, multiple myeloma, mantle cell lymphoma, acute lymphatic leukemia, chronic lymphatic leukemia, B-cell lymphoma, T-cell lymphoma, Hodgkin's lymphoma, non-Hodgkin's lymphoma, hairy cell lymphoma).

### BRIEF DESCRIPTION OF THE DRAWINGS

- Figure 1:: Electron microscopic image of the material obtained from example 2
- Figure 2:: Electron microscopic image of the material obtained from reference example 1
- Figure 3:: Electron microscopic image of the material obtained from reference example 2
- Figure 4:: Dissolution profiles (% vs. minutes) at pH 4.5 of pharmaceutical compositions of example 4 prepared with palbociclib having low (A), medium (B) and high (C) specific surface area.

### DETAILED DESCRIPTION OF THE INVENTION

The invention is described below in further detail by embodiments, without being limited. The invention may be characterized by one or more of the following embodiments or by combining two or more of them.

The invention relates to crystalline free base of palbociclib characterized by having a specific surface area in the range of from about 2.1 to 6.0 m²/g, preferably from about 2.3 to 5.5 m²/g, more preferably from about 2.5 to 5.0 m²/g, even more preferably from about 2.5 to 4.8 m²/g, even more preferably from about 2.5 to 4.5 m²/g, even more preferably from about 2.8 to 4.5 m²/g, even more preferably from about 3.0 to 4.5 m²/g, even more preferably from about 3.0 to 4.3 m²/g, even more preferably from about 3.0 to 4.0 m²/g, even more preferably from about 3.0 to 3.8 m²/g, and most preferably from about 3.0 to 3.5 m²/g, such as from about 3.0 to 3.3 m²/g as determined by BET-nitrogen adsorption analysis. Each of the values of the lower limit of such ranges can be combined with any value of the upper limit of such ranges that is not inconsistent with it.

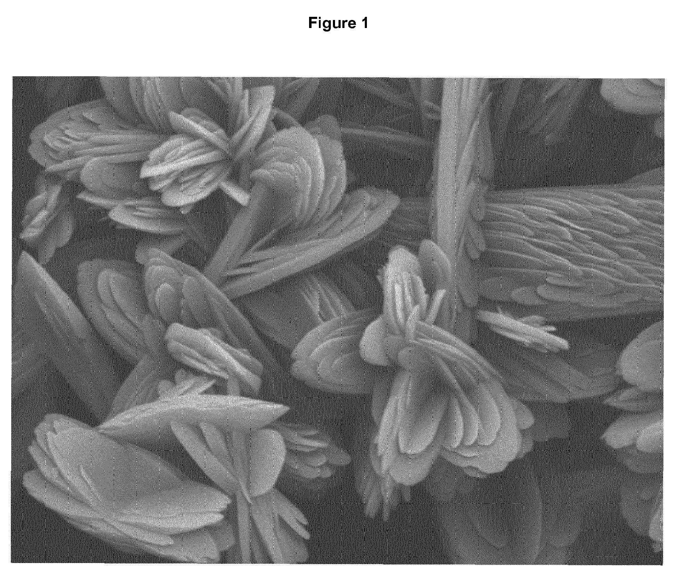

Palbociclib free base of the invention is also characterized by having flake-like and/or plate-like primary particles, preferably rounded flake-like and/or plate-like primary particles, which have indistinct corners and edges. The palbociclib base of the invention preferably contains at least 50%, more preferably at least 60%, even more preferably at least 70%, even more preferably at least 80%, even more preferably at least 90% primary particles having flake-like and/or plate-like morphology. Most preferably the palbociclib base of the invention consists of primary particles having flake-like and/or plate-like morphology. A characteristic electron microscopic image is displayed in figure 1 herein.

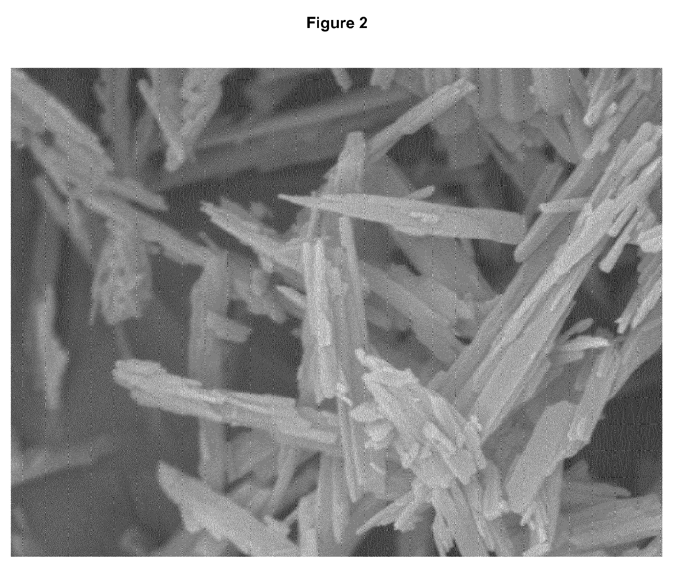

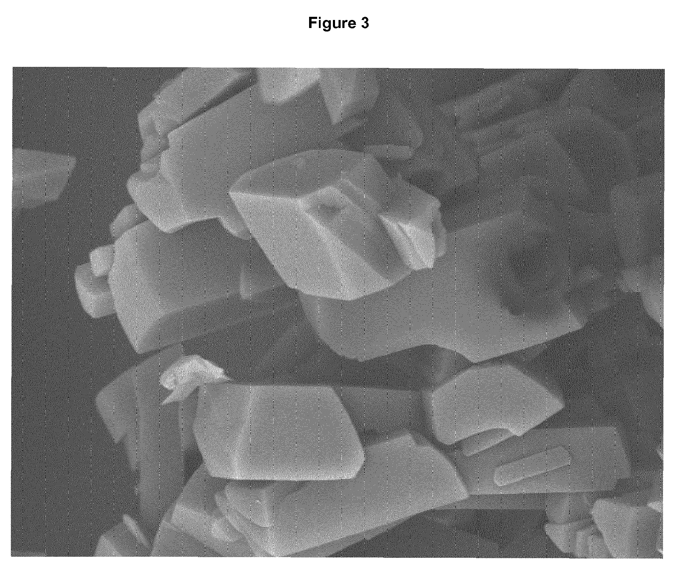

In contrast, palbociclib free base obtained by the method disclosed in WO 2005/005426 A1 consists of needle-like primary particles. A characteristic electron microscopic image is displayed in figure 2 herein. On the other hand, palbociclib free base obtained by the method disclosed in WO 2014/128588 A1 consists of blocky equant and columnar primary particles. A characteristic electron microscopic image is displayed in figure 3 herein. More preferably the palbociclib of the invention is substantially free from needle-like or blocky equant or columnar primary particles. Most preferably, the palbociclib of the invention is substantially free from needle-like and blocky equant and columnar primary particles.

The shape of a particle influences the pharmaceutical performance of a material, such as bulk and tap density, processability, tableting behavior, compaction, compressibility, flowability, tendency to stick to the punches or dissolution properties.

Preferably, the crystalline free base of palbociclib of the invention is characterized by having a PXRD comprising a reflection at (22.5 ± 0.2)° 2-Theta, when measured at room temperature with Cu-Kalpha_{1,2} radiation having a wavelength of 0.15419 nm.

More preferably, the crystalline free base of palbociclib of the invention is characterized by having a PXRD comprising reflections at 2-Theta angles of (10.3 ± 0.2)°, (22.5 ± 0.2)°, when measured at room temperature with Cu-Kalpha_{1,2} radiation having a wavelength of 0.15419 nm.

More preferably, the crystalline free base of palbociclib of the invention is characterized by having a PXRD comprising reflections at 2-Theta angles of (5.1 ± 0.2)°, (10.3 ± 0.2)°, (22.5 ± 0.2)°, when measured at room temperature with Cu-Kalpha_{1,2} radiation having a wavelength of 0.15419 nm.

More preferably, the crystalline free base of palbociclib of the invention is characterized by having a PXRD comprising reflections at 2-Theta angles of (5.1 ± 0.2)°, (8.0 ± 0.2)°, (10.3 ± 0.2)°, (22.5 ± 0.2)°, when measured at room temperature with Cu-Kalpha_{1,2} radiation having a wavelength of 0.15419 nm.

Even more preferably, the crystalline free base of palbociclib of the present invention is characterized by having a PXRD comprising reflections at 2-Theta angles of (5.1 ± 0.2)°, (8.0 ± 0.2)°, (10.3 ± 0.2)°, (11.5 ± 0.2)°, (22.5 ± 0.2)°, when measured at room temperature with Cu-Kalpha_{1,2} radiation having a wavelength of 0.15419 nm.

Most preferably, the crystalline free base of palbociclib of the present invention is characterized by having a PXRD comprising reflections at 2-Theta angles of (5.1 ± 0.2)°, (8.0 ± 0.2)°, (10.3 ± 0.2)°, (11.5 ± 0.2)°, (22.5 ± 0.2)° and at least one additional reflection selected from 2-Theta angles of (17.1 ± 0.2)°, (18.8 ± 0.2)°, (19.7 ± 0.2)°, when measured at room temperature with Cu-Kalpha_{1,2} radiation having a wavelength of 0.15419 nm. Alternatively, the crystalline free base of palbociclib of the invention is characterized by showing a PXRD essentially the same as displayed in figure 1 of WO 2014/128588 (Form A), when measured at room temperature with Cu-Kalpha_{1,2} radiation having a wavelength of 0.15419 nm.

Furthermore, the invention relates to pharmaceutical compositions comprising an effective amount of the crystalline palbociclib free base of the invention and at least one pharmaceutically acceptable excipient.

Preferably, the effective amount of the crystalline palbociclib free base of the invention is in the range of from about 10 to 200 mg, more preferably from about 25 to 150 mg, even more preferably from about 50 to 130 mg and most preferably the effective amount of crystalline palbociclib free base of the invention is about 75, 100 or 125 mg.

Pharmaceutically acceptable excipients which may be used for the pharmaceutical compositions of the invention are preferably selected from the group consisting of fillers, diluents, sweeteners, buffering agents, glidants, flavouring agents, lubricants, preservatives, surfactants, wetting agents, binders, disintegrants, colouring agents and thickeners. Other excipients known to be used for the preparation of pharmaceutical compositions may also be used.

Preferably, pharmaceutical compositions of the invention comprise an effective amount of palbociclib free base of the invention and one or more pharmaceutically acceptable excipients selected from the group of fillers, diluents, disintegrants, glidants and lubricants.

More preferably, pharmaceutical compositions of the invention comprise an effective amount of palbociclib free base of the invention and one or more pharmaceutically acceptable excipients selected from microcrystalline cellulose, lactose monohydrate, sodium starch glycolate, colloidal silicon dioxide and magnesium stearate.

Pharmaceutical compositions of the invention comprising an effective amount of crystalline palbociclib free base of the invention are preferably oral dosage forms, more preferably tablets or capsules and most preferably capsules.

Furthermore, the invention relates to pharmaceutical compositions comprising an effective amount of palbociclib and one or more pH modifier in order to ensure a low pH in the stomach. This surprisingly leads to an improved dissolution profile and improved bioavailability independent of the pH in the stomach. Also variability is significantly reduced and malabsorptions are prevented. Particularly good dissolution and bioavailability are obtained when the medium specific surface area particles of the invention are combined with a pH modifier.

The pH modifier is preferably a weak acid, more preferably an organic acid. More preferably, the pH modifier is selected from the group consisting of succinic acid, tartaric acid, ascorbic acid, citric acid, lactic acid, malic acid, fumaric acid, aspartic acid, glutamic acid, hydrates, salts and mixtures thereof. Most preferably the pH modifier is succinic acid or a mixture of succinic and tartaric acid.

The pH modifier is preferably used in an amount between 1 and 40% w/w, more preferably between 2 and 30% w/w, most preferably between 2 and 20% w/w on the total weight of the pharmaceutical composition.

The pharmaceutical compositions of the invention have a dissolution at pH = 1 of at least 70%, preferably of 80%, most preferably of 85% within 15 minutes.

In addition, the invention relates to pharmaceutical compositions comprising an effective amount of crystalline palbociclib free base of the invention for use as medicament.

In particular, the invention relates to pharmaceutical compositions comprising an effective amount of crystalline palbociclib free base of the invention for use in the treatment of cancer.

Moreover, the present invention relates to a process for the preparation of palbociclib free base comprising:
(a) providing an acid addition salt of palbociclib;
(b) preparing a suspension comprising the acid addition salt provided in (a) and a chlorinated solvent;
(c) treating the suspension prepared in (b) with an amine base;
(d) extracting the solution obtained in (c) with water or an aqueous salt solution;
(e) subjecting the organic phase obtained in (d) to crystallization conditions comprising one or more of the following steps:
   (e.1) seeding with crystalline palbociclib free base;
   (e.2) at least partially removing the solvent;
   (e.3) combining the solution with an antisolvent;
(f) optionally separating at least a part of the crystalline palbociclib free base from its mother liquor;
(g) optionally drying the crystals obtained in (f);

Suitable acid addition salts which may be provided in step (a) are selected from acid addition salts of palbociclib with inorganic acids such as hydrochloric acid, hydrobromic acid, sulfuric acid and nitric acid, from acid addition salts of palbociclib with organic acids such as isethionic acid, methanesulfonic acid, ethane-1,2-disulfonic acid, naphthalene-1,5-disulfonic acid, p-toluenesulfonic, thiocyanic acid, cyclamic acid and trifluoroacetic acid or mixtures thereof. Acid addition salts of palbociclib may be employed as mono-salts having approximately 1:1 stoichiometry such as palbociclib monohydrochloride or as di-salts having approximately 1:2 stoichiometry such as palbociclib dihydrochloride. Preferably, palbociclib monohydrochloride, palbociclib dihydrochloride or mixtures thereof and most preferably palbociclib dihydrochloride is used as starting material in the process of the invention. The acid addition salts may be prepared in accordance with the procedures disclosed in WO 2003/062236 A1, WO 2005/005426 A1 or WO 2008/032157 A2. In general, the aforementioned acid addition salts of palbociclib may be prepared by treating palbociclib free base with the corresponding acid in the presence of a suitable solvent.

One or more of the aforementioned acid addition salts of palbociclib is then suspended in a solvent comprising a chlorinated solvent. A chlorinated solvent is a solvent that contains at least one chlorine atom. Preferred chlorinated solvents are dichloromethane, chloroform, chlorobenzene, 1,2-dichlorobenzene, 1,3-dichlorobenzene, 1,4-dichlorobenzene and mixture thereof. Most preferably the chlorinated solvent is dichloromethane. The palbociclib acid addition salt concentration of the suspension is preferably in the range of from about 5 to 60 g, more preferably from about 15 to 40 g per liter solvent. The suspension is preferably prepared at a temperature of about 40 °C or less, more preferably of about 25 °C or less, for example at about room temperature.

The obtained suspension is then treated with an amine base by adding the amine base to the suspension or *vice versa*. Suitable amine bases which may be employed are preferably selected from ammonia, methylamine, ethylamine, cyclohexylamine, dimethylamine, diethylamine, trimethylamine, triethylamine, diisopropylethylamine or mixtures thereof, more preferably from ammonia, trimethylamine, diisopropylethylamine or mixtures thereof. Most preferably, the suspension is treated with triethylamine. The molar ratio of palbociclib acid addition salt and amine base applied is preferably in the range of from about 1 : 2 to 12, more preferably from about 1 : 4 to 8. Preferably, the amount of base is chosen such that at the end of the acid base reaction a solution, preferably a clear solution, more preferably a clear solution free of any solid material is obtained. The acid base reaction is preferably carried out at a temperature of about 40 °C or less, more preferably of about 25 °C or less, for example the reaction is performed at about room temperature.

The obtained solution is then extracted water or an aqueous salt solution. Preferably, the aqueous salt solution is an aqueous sodium chloride solution. Extracting may comprise stirring and/or shaking the product solution obtained in (c) with one or more of the aforementioned aqueous solvents. By means of extracting using an aqueous solvent, water soluble amine salts, which are present in the solution obtained from step (c) are depleted or even removed. Optionally the aqueous phase may be extracted with dichloromethane before discarding the aqueous layer. The combined dichloromethane phases are then optionally diluted with further dichloromethane before subjecting the solution to crystallization conditions.

The product solution obtained in step (d) is subjected to crystallization conditions comprising one or more of the following steps:
(e.1) seeding with crystalline palbociclib free base;
(e.2) at least partially removing the solvent;
(e.3) combining the solution with an antisolvent;

The amount of seed crystals employed in step (e.1) may be in the range of from about 1 to 20% (w/w), preferably from about 1 to 10% (w/w), more preferably from about 1 to 5% (w/w) and most preferably from about 1 to 2% (w/w) based on the amount of palbociclib free base present in the product solution. Any crystalline form of palbociclib free base may be applied as seeds, whereat most preferably crystalline form A of palbociclib is used for seeding.

In step (e.2) the volume of the solution is reduced by solvent evaporation. The solvent may either be completely removed or reduced to an extent in the range of from about 5 to 99% (v/v), preferably from about 30 to 70% (v/v) based on the volume of the initial solution. The solvent may be preferably evaporated at a temperature in the range of from about 25 to 60 °C. Solvent evaporation may be performed at reduced or at atmospheric pressure.

In step (e.3) the chlorinated solvent solution is combined with an antisolvent, i.e. a liquid miscible with the solvent which reduces solute solubility in this new mixed solvent. The antisolvent is preferably selected from *n*-heptane, cyclohexane, *tert*-butylmethylether, diisopropylether or mixtures thereof, whereat most preferably *n*-heptane is used as antisolvent. Thereby, the antisolvent may be added to the dichloromethane solution or *vice versa.* The antisolvent/solvent ratio applied may be in the range of from about 1 : 10 (v/v), preferably from about 1 : 8 (v/v), more preferably from about 1 : 6 (v/v), even more preferably from about 1 : 4 (v/v) and most preferably from about 1 : 2 (v/v).

Crystallization may be initiated by subjecting the product solution obtained in step (d) to one or more of steps e.1) to (e.3) in any order.

Subsequently, the obtained palbociclib crystals may be separated from their mother liquor by any conventional method such as filtration, centrifugation, evaporation of the solvent, decantation of the solvent or by two or more of these methods. Most preferably, the palbociclib crystals are collected by filtration.

Finally, the isolated palbociclib crystals may be dried at a temperature of about 100 °C or less, preferably at a temperature of about 40 °C or less, for example at about room temperature. Drying may be performed at atmospheric or at reduced pressure for a period of about 168 hours or less.

WO 2014/128588 A1 teaches that palbociclib free base provided by salt break procedures, e.g. as described in example 4 of WO 2005/005426 A1, was highly static prone and formed small primary particles, which agglomerated into large, hard agglomerates that were difficult to disperse by sieving and were unsuitable for further development (see page 2, lines 4 to 7). Therefore, WO 2014/128588 A1 provides palbociclib having large primary particles and greatly reduced specific surface area which, according to the applicant, demonstrates improved physicochemical and manufacturability properties.

It was surprisingly found that according to the modified salt break procedure of the present invention crystalline palbociclib free base having medium specific surface area is obtained. The modified salt break procedure of the present invention gives high yields of crystalline palbociclib free base having a specific surface area in the range of from about 2.1 to 6.0 m²/g, preferably from about 2.5 to 5.0 m²/g, more preferably from about 2.5 to 4.5 m²/g, even more preferably from about 3.0 to 4.5 m²/g, even more preferably from about 3.0 to 4.0 m²/g and most preferably from about 3.0 to 3.5 m²/g, such as from about 3.0 to 3.3 m²/g as determined by BET-nitrogen adsorption analysis. Furthermore, the obtained material is characterized by having flake-like and plate-like primary particles, preferably rounded flake-like and plate-like primary particles, which have indistinct corners and edges.

Table 1 provides a comparison of specific surface areas and particle shapes of crystalline palbociclib free base prepared according to different methods.

**Table 1: Specific surface areas and particle shape of palbociclib prepared according to different methods**

| **Palbociclib free base prepared according to** | **BET SSA by N₂** | **particle shape** |
|---|---|---|
| | **[m²/g]** | |
| salt break procedure of WO 2005/005426 (data from table 12 of WO 2014/128588 A1) | | needles |
| | 6.6 | |
| modified salt break procedure of the present invention (data from examples 1, 2 and 3 herein) | 3.0 | flakes/ plates |
| | 3.3 | |
| | 4.5 | |
| method disclosed in WO 2014/128588 A1 (data from table 12 of WO2014/128588 A1) | 0.6 | equant/ columns |
| | 0.7 | |
| | 0.7 | |

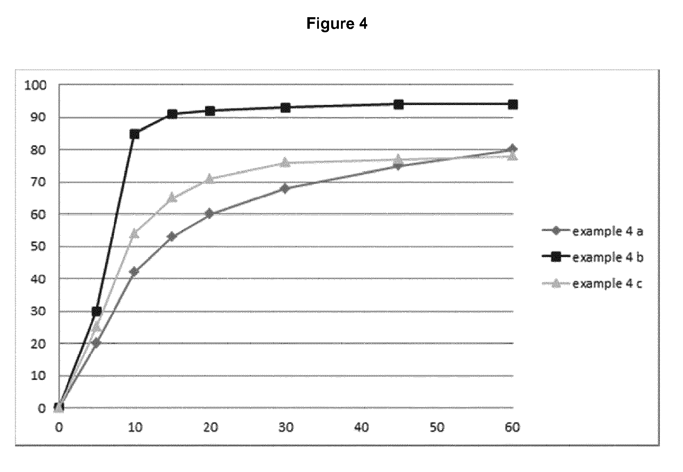

In contrast to palbociclib free base having either greatly reduced or huge specific surface area, crystalline palbociclib of the present invention is characterized by having intermediate specific surface area. This material on the one hand demonstrates viable physicochemical and manufacturability properties, such as good flowability, no electrostatic behavior, nor adherence to the laboratory and production equipment, which allow for straight forward pharmaceutical processing and on the other hand shows faster dissolution compared to the coarse material obtained according to the procedure disclosed in WO 2014/128588 A1, which ensures optimal bioavailability. This is particularly evident when the dissolution profile is measured at pH 4.5 (see Figure 4) and proves particularly advantageous in patients having a pH of the stomach higher than the normal pH.

In addition, due to the fact that the Palbociclib particles of the invention have a size comparable to that of the other excipients, a more homogeneous mixture, showing no sedimentation or separation, is obtained, which provides for more uniformity of the final dosage form. These effects are additionally promoted by the flake-like and plate-like crystal habit of the material. Therefore, crystalline palbociclib free base having medium specific surface area and flake-like respectively plate-like primary particles as provided by the present invention is preferably used for the preparation of orally administered pharmaceutical compositions.

In addition, according to the modified salt break procedure of the invention palbociclib is obtained in high yield and purity and with improved filterability.

The following non-limiting examples are illustrative for the disclosure.

### EXAMPLES

PXRDs were obtained with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. The diffractograms were recorded at a tube voltage of 45 kV and a tube current of 40 mA, applying a stepsize of 0.013° 2-Theta with 40s per step (255 channels) in the angular range of 2° to 40° 2-Theta at room temperature. A typical precision of the 2-Theta values is in the range of ± 0.2° 2-Theta. Thus, for example the diffraction peak of Form A that appears at 5.1° 2-Theta can appear between 4.9 and 5.3° 2-Theta on most X-ray diffractometers under standard conditions.

Specific surface area measurements (BET Nitrogen) were performed using a Quantachrome Instruments Nova 2000e (Vers.: 9.01) specific surface area analyzer. The following setup has been used for the measurement:

| | |
|---|---|
| Software version: | NovaWin Version 9.0 |
| Adsorbate: | Nitrogen |
| Sample tube: | 9 mm cell (small globe) with glass filler rods |
| Sample mass: | 0.13 - 0.21 g |
| Out gassing conditions: | 1 h at 50 °C under vacuum (heating mantles) |
| Isothermal collection points: | 5 point BET in the range 0.01-0.30 P/Po |
| Isothermal data analysis range: | 5 point BET in the range 0.01-0.30 P/Po |
| Sample volume: | Calculated |
| Po option: | Calculated |
| Thermal delay: | 180 s |
| Equilibration time: | 60 s |
| Equilibration timeout: | 240 s |
| | |
| Sample Density: 1.313 g/cm³ | |

Dissolution profiles were obtained using a USP II apparatus with a sinker at 50 rpm and 1000 ml of 0.1 M hydrochloric acid (pH 1) or 1000 ml of 0,05 M acetate buffer (pH 4,5).

### Reference Example 1: Preparation of palbociclib free base according to the method of example 5 of WO 2014/128588

Palbociclib dihydrochloride (17.5 g) was dissolved in 210 mL of de-ionized water and 122.5 mL of acetone by heating to a temperature of 50 °C. The obtained solution was further stirred at 50 °C for about 75 min before cooling it to 35 °C in 30 min. Thereafter, the solution was treated in 5 mL portions with a total of 55 mL of 5% (w/w) aqueous sodium hydroxide solution until a pH of 12.4 was achieved, whereupon crystallization was observed. Subsequently, the obtained suspension was cooled to 22.5 °C in 15 min and stirred at this temperature for 1 h. Finally, the crystals were collected by filtration and washed with 50 mL of de-ionized water and 50 mL of acetone before they were dried at RT under vacuum (20-30 mbar) for 19 h. This material was equivalent to the material prepared by treatment of palbociclib hydrochloride with aqueous sodium hydroxide as in example 4 of WO 2005/005426 A1.

Yield: 13.7 g (91% of theory)

### Reference Example 2: Preparation of palbociclib free base according to the method of WO 2014/128588 A1

Palbociclib free base (10.0 g, e.g. prepared according to the procedure described in example 4 of WO 2005/005426 A1) was suspended in 160 mL of n-butanol and 240 mL of anisole and heated with stirring between 95 and 100 °C. The obtained solution was then cooled to 80 °C and seeded with a suspension consisting of 50 mg free base in 2.5 mL n-butanol. The resulting slurry was stirred at 80 °C for 3 h before cooling to 10°C over 350 min (cooling rate of 0.2 K/min) and further stirring the suspension at this temperature for 10 min. Thereafter, the solid was collected by filtration and the cake washed with anisole (50 mL) and n-heptane (50 mL) before drying it under vacuum at room temperature for 18 h.

Yield: 7.5 g (75% of theory)

### Example 1: Preparation of palbociclib free base having medium specific surface area - 25 g scale

A suspension of palbociclib dihydrochloride (25.0 g, 48.0 mmol, prepared according to the procedure disclosed in example 36 of WO 2003/062236 A1) in methylene chloride (960 mL) was treated with triethylamine (44.5 mL, 321.0 mmol) at RT. The obtained clear solution was extracted with 600 mL deionized water. Thereafter, the phases were separated and the aqueous phase was extracted with 600 mL methylene chloride and discarded. To the combined methylene chloride phases additional methylene chloride (960 mL) was added before reducing the volume to about 900 mL by distillation (T_{bath} = 53 °C, atmospheric pressure). The meanwhile slightly hazy solution was allowed to cool to 31 ± 1 °C before n-heptane (960 mL) was added speedily under stirring which initiated crystallization. The obtained suspension was further stirred at RT for 17 h. Finally, the crystals were collected by filtration and dried at RT under vacuum (20-30 mbar) for 6 h.

Yield: 20.1 g (94% of theory); SSA: 3.3 m²/g, PXRD: Form A

### Example 2: Preparation of palbociclib free base having medium specific surface area - 20 g scale

A suspension of palbociclib dihydrochloride (20.0 g, 38.4 mmol, prepared according to the procedure disclosed in example 36 of WO 2003/062236 A1) in methylene chloride (800 mL) was treated with triethylamine (37.1 mL, 267.6 mmol) at RT. The obtained clear solution was extracted with 500 mL deionized water. Thereafter, the phases were separated and the aqueous phase was extracted with 500 mL methylene chloride and discarded. To the combined methylene chloride phases additional methylene chloride (800 mL) was added before reducing the volume to about 750 mL by distillation (T_{bath} = 53 °C, atmospheric pressure). The meanwhile slightly hazy solution was allowed to cool to 30 ± 1 °C before n-heptane (960 mL) was added speedily under stirring which initiated crystallization. The obtained suspension was further stirred at RT for 6 h. Finally, the crystals were collected by filtration and dried at RT under vacuum (20-30 mbar) for about 65 h.

Yield: 16.0 g (93% of theory); SSA: 3.0 m²/g, PXRD: Form A

### Example 3: Preparation of palbociclib free base having medium specific surface area - 2 g scale

A suspension of palbociclib dihydrochloride (2.0 g, 3.8 mmol, prepared according to the procedure disclosed in example 36 of WO 2003/062236 A1) in methylene chloride (80 mL) was treated with triethylamine (3.7 mL, 26.7 mmol) at RT. The obtained clear solution was extracted with 50 mL deionized water. Thereafter, the phases were separated and the aqueous phase was extracted with 50 mL methylene chloride and discarded. To the combined methylene chloride phases additional methylene chloride (80 mL) was added before reducing the volume to about 100 mL by distillation (T_{bath} = 53 °C, atmospheric pressure). The meanwhile slightly hazy solution was allowed to cool to 30 ± 1 °C before n-heptane (80 mL) was added speedily under stirring which initiated crystallization. The obtained suspension was further kept at RT without stirring for 2 h. Finally, the crystals were collected by filtration and dried at RT under vacuum (20-30 mbar) for 17 h.

Yield: 1.6 g (93% of theory); SSA: 4.5 m²/g, PXRD: Form A

### Example 4: formulations

| | **A** | **B** | **C** |
|---|---|---|---|
| Palbociclib | 75 mg | 75 mg | 75 mg |
| (source) | (Ref. Ex. 2) | (Ex. 1) | (Ref. Ex. 1) |
| Microcrystalline cellulose | 36.4 mg | 36.4 mg | 36.4 mg |
| Lactose | 36.4 mg | 36.4 mg | 36.4 mg |
| SiO₂ | 0.4 mg | 0.4 mg | 0.4 mg |
| Magnesium stearate | 0.8 mg | 0.8 mg | 0.8 mg |
| Sodium starch glycolate | 1.1 mg | 1.1 mg | 1.1 mg |

A mixture of the crystalline palbociclib of the invention (medium specific surface area), microcrystalline cellulose, lactose monohydrate, sodium starch glycolate, colloidal silicon dioxide and magnesium stearate is made and filled into capsules.

The dissolution profiles for the 3 formulations have been measured at pH 4.5 and are reported in Figure 4 and in the following table:

| min | A | B | C |
|---|---|---|---|
| 0 | 0 | 0 | 0 |
| 5 | 20 | 30 | 25 |
| 10 | 42 | 85 | 54 |
| 15 | 53 | 91 | 65 |
| 20 | 60 | 92 | 71 |
| 30 | 68 | 93 | 76 |
| 45 | 75 | 94 | 77 |
| 60 | 80 | 94 | 78 |

Surprisingly, formulation B, comprising the palbociclib particles of the invention, shows a markedly better dissolution than formulations A and C.

Alternatively, a mixture of the crystalline palbociclib of the invention (medium specific surface area), microcrystalline cellulose, lactose monohydrate and sodium starch glycolate is made and granulated in a fluid bed. Colloidal silicon dioxide and magnesium stearate are added and mixed. Afterwards a tablet is compressed.

### Example 5: formulations

| | **A** | **B** |
|---|---|---|
| Palbociclib | 75 mg | 75 mg |
| Mannitol | 74.6 mg | 99.5 mg |
| SiO₂ | 0.4 mg | 0.5 mg |

A mixture of the crystalline palbociclib of the invention (medium specific surface area), mannitol and colloidal silicon dioxide is made and filled into capsules.

### Example 6: formulations with pH modifier

| | **A** | **B** | **C** | **D** |
|---|---|---|---|---|
| Palbociclib | 75 mg | 75 mg | 75 mg | 75 mg |
| Succinic acid | 30 mg | | 20 mg | |
| Tartaric acid | | | 20 mg | |
| Ascorbic acid | | 30 mg | | 30 mg |
| Mannitol | 59.5 mg | 73.6 mg | 59.5 mg | 73.6 mg |
| SiO₂ | 0.5 mg | 0.4 mg | 0.5 mg | 0.4 mg |

A mixture of the crystalline palbociclib of the invention (medium specific surface area), pH modifier, mannitol and colloidal silicon dioxide is made and filled into capsules.

### Example 7: formulations with pH modifier

A mixture of palbociclib, pH modifier, microcrystalline cellulose, lactose monohydrate, sodium starch glycolate, colloidal silicon dioxide and magnesium stearate is made and filled into the capsules.

| | **A** | **B** | **C** | **D** |
|---|---|---|---|---|
| Palbociclib | 75 mg | 75 mg | 75 mg | 75 mg |
| Succinic acid | 20 mg | | 20 mg | |
| Tartaric acid | | 10 mg | 20 mg | |
| Ascorbic acid | | | | 5 mg |
| Microcrystalline cellulose | 35.8 mg | 28.5 mg | 35.8 mg | 28.5 mg |
| Lactose | 35.9 mg | 28.5 mg | 35.9 mg | 28.5 mg |
| SiO₂ | 0.4 mg | 0.5 mg | 0.4 mg | 0.5 mg |
| Magnesium stearate | 0.8 mg | 1.0 mg | 0.8 mg | 1.0 mg |
| Sodium starch glycolate | 1.1 mg | 1.5 mg | 1.1 mg | 1.5 mg |

### Example 8: formulations

| | **A** | **B** | **C** | **D** |
|---|---|---|---|---|
| Palbociclib | 75 mg | 75 mg | 75 mg | 75 mg |
| Succinic acid | 20 mg | 20 mg | | |
| Lauroyl macrogol-32 glycerides | 50 mg | 100 mg | | |
| Stearoyl macrogol-32 glycerides | | | 50 mg | 100 mg |

Palbociclib and succinic acid are melt in the given amount of lauroyl macrogol-32 glycerides or stearoyl macrogol-32 glycerides and the mixture is filled into capsules.

### Example 9: pellets formulation

| | **A** | **B** |
|---|---|---|
| Palbociclib | 75 mg | 75 mg |
| Tartaric acid pellets | 35 mg | 45 mg |
| Hydroxypropyl cellulose | 9 mg | |
| Hydroxypropyl methylcellulose | | 11 mg |

Hydroxypropyl cellulose or hydroxypropyl methylcellulose is dissolved in isopropanol until a clear solution is obtained. Palbociclib is then added and the solution is coated onto the tartaric acid pellets. After drying the pellets are filled into the capsules. Alternatively the palbociclib solution can be sprayed onto the pellets.

## Claims

1. Crystalline palbociclib having a specific surface area in the range of from 2.1 to 6.0 m²/g as determined by BET-nitrogen adsorption analysis.

2. The crystalline palbociclib according to claim 1, **characterized by** having a specific surface area in the range of from 2.5 to 5.0 m²/g as determined by BET-nitrogen adsorption analysis.

3. The crystalline palbociclib according to claim 1 or 2, **characterized by** having a specific surface area in the range of from 3.0 to 4.5 m²/g as determined by BET-nitrogen adsorption analysis.

4. The crystalline palbociclib according to any one of the preceding claims having flake-like and/or plate-like primary particles.

5. Crystalline palbociclib having flake-like and/or plate-like primary particles.

6. The crystalline palbociclib according to any one of the preceding claims having a powder X-ray diffractogram comprising reflections at 2-Theta angles of (5.1 ± 0.2)°, (10.3 ± 0.2)°, (22.5 ± 0.2)°, when measured at a temperature in the range of from 15 to 25 °C with Cu-Kalpha_{1,2} radiation having a wavelength of 0.15419 nm.

7. A process for the preparation of crystalline palbociclib according to any one of claims 1 to 6 comprising:
(a) providing an acid addition salt of palbociclib;
(b) preparing a suspension comprising the acid addition salt provided in (a) and a chlorinated solvent;
(c) treating the suspension prepared in (b) with an amine base;
(d) extracting the solution obtained in (c) with water or an aqueous salt solution;
(e) subjecting the organic phase obtained in (d) to crystallization conditions comprising one or more of the following steps:
(e.1) seeding with crystalline palbociclib free base;
(e.2) at least partially removing the solvent;
(e.3) combining the solution with an antisolvent;

8. The process of claim 7 further comprising separating at least a part of the crystalline base of palbociclib from its mother liquor.

9. The process according to claim 7 to 8 wherein the chlorinated solvent is dichloromethane.

10. Crystalline palbociclib obtainable by the process according to any one of claims 7 to 9.

11. A pharmaceutical composition comprising an effective amount of palbociclib and a pH modifier.

12. The pharmaceutical composition according to claim 11 comprising the palbociclib of claims 1 to 6 or 10.

13. The pharmaceutical composition according to claim 11 or 12 wherein the pH modifier is a weak acid, preferably an organic acid.

14. The crystalline palbociclib according to any one of claims 1 to 6 or 10 or the pharmaceutical composition according to claims 11 to 13 for use as medicament.

15. The crystalline palbociclib according to any one of claims 1 to 5 or 10 or the pharmaceutical composition according to claims 11 to 13 for use in the treatment of cancer.
